# FASCICULE DE BREVET EUROPEEN

(11) **EP 2 616 014 B1**
(45) Date de publication et mention de la délivrance du brevet: **14.05.2014**
(21) Numéro de dépôt: 11773090.3
(22) Date de dépôt: 15.09.2011
(51) Int. Cl.: A61F 2/40

(54) **CUPULE MODULABLE DE PROTHÈSE D'ÉPAULE**
ADAPTIERBARE SCHULTERPROTHESENPFANNE
ADAPTABLE SHOULDER PROSTHESIS CUP

(30) Priorité: 17.09.2010 FR 1057436
(43) Date de publication de la demande: 24.07.2013
(73) Titulaire: Duport, Marc, 31400 Toulouse (FR)
(72) Inventeur: Duport, Marc, 31400 Toulouse (FR)
(74) Mandataire: Jeannet, Olivier
(86) Numéro de dépôt international: PCT/FR2011/052116
(87) Numéro de publication internationale: WO 2012/035264

(56) Documents cités:
- EP-A2- 0 538 895
- WO-A2-2008/146124
- FR-A1- 2 686 503
- FR-A1- 2 928 827
- US-A1- 2003 163 202
- US-A1- 2004 153 161
- US-A1- 2010 049 329

## Description

La présente invention se rapporte à une cupule de prothèse, notamment une cupule de prothèse d'épaule.

En France, environ 15 000 prothèses d'épaule sont posées par an, incluant les prothèses totales d'épaules, dites standards, les prothèses inversées, et plus récemment les prothèses de resurfaçage.

Ce chiffre, en nette augmentation ces dernières années, est en particulier dû à l'augmentation du nombre de pathologies pour lesquelles la pose d'une prothèse d'épaule est maintenant indiquée.

Dans ce contexte, la chirurgie prothétique de l'épaule reste une chirurgie très spécialisée, de haute technicité, nécessitant un apprentissage long et l'utilisation d'ancillaires souvent complexes. Une simplification des prothèses et des ancillaires est donc souhaitable afin de faciliter le geste opératoire, de le rendre plus reproductible et donc de diminuer les complications postopératoires. Une telle simplification de l'ancillaire et du geste opératoire aurait également l'avantage de diminuer les coûts de ces opérations de pose de prothèses d'épaule.

Il existe plusieurs types de prothèses d'épaule. Certaines prothèses classiques telles que celle décrite dans US 2004/153161 sont fixées sur l'humérus, grâce à une tige humérale, après une résection conséquente de l'os. L'implantation d'une tige humérale nécessite un évidement important dans l'os pour l'accueillir, diminuant ainsi le capital osseux et fragilisant l'os. La pose d'une telle prothèse est longue et complexe. Après installation de la tige dans l'évidement, l'espace entre l'os et la tige humérale est généralement comblé avec du ciment. Avec le temps, le ciment peut se disloquer et ne plus assurer alors une fixation correcte de la tige.

Il existe également des prothèses de resurfaçage, telles que celle décrite dans FR 2 928 827, qui sont posées sur l'épiphyse de l'humérus après un éventuel ponçage léger de la surface de l'épiphyse. De telles prothèses ne sont pas nécessairement ancrées dans l'os comme décrit précédemment.

Lorsqu'un simple resurfaçage de l'épiphyse n'est pas indiqué, par exemple en cas de fragilité osseuse trop importante, le chirurgien peut choisir de mettre en oeuvre une technique intermédiaire dite de « demi-resurfaçage » ou une partie de l'épiphyse est réséquée. Le chirurgien est alors amené à utiliser une prothèse telle que celle décrite dans le document EP 0538 895, comportant une cupule destinée à remplacer la partie de l'épiphyse réséquée et une vis d'ancrage assure la fixation de la cupule sur l'os.

Le document US 2003/0163202 décrit une cupule comportant un plot central de fixation.

Il est également connu du document WO 2008/146124 de remplacer la vis d'ancrage décrite précédemment par une quille d'ancrage sensiblement cylindrique. La cupule est alors pourvue d'un plot d'accouplement coopérant par emboitement avec une cavité, de forme cylindrique complémentaire, ménagée à cet effet dans la quille d'ancrage. Une telle cupule est alors maintenue en place grâce à la pression exercée par la glène. Cependant, la nature de l'accouplement de la cupule et de la quille d'ancrage ne permet pas d'éviter la rotation de la cupule après son implantation, ce qui peut occasionner des problèmes. De plus, la cupule étant simplement emboitée dans la cavité de la quille d'ancrage, l'ancrage de la cupule sur la tête humérale peut s'avérer insuffisant, et ainsi occasionner des douleurs ou un inconfort pour le patient.

Le document FR 2 686 503 décrit une prothèse de réhabilitation d'une tête fémorale, comportant une cupule équipée d'un plot d'ancrage central dans l'os, entouré de plusieurs picots qui en sont parallèles et sont destinés à pénétrer dans l'os du fémur pour améliorer la fixation de la cupule.

Le préambule de la revendication 1 est basé sur ce que décrit ce document.

Toutes les cupules connues sont spécifiquement adaptées à une technique chirurgicale précise. Le chirurgien doit donc apprendre les différentes techniques et doit disposer de plusieurs ancillaires adaptés à chaque technique. Par ailleurs, si au cours d'une opération le chirurgien rencontre des difficultés, il n'a pas la possibilité d'adapter la prothèse, par exemple en changeant la méthode d'implantation de la cupule.

La présente invention a pour objet de remédier en tout ou partie aux différents inconvénients cités précédemment.

Dans ce contexte, la présente invention a pour objet de proposer une cupule modulable permettant la mise en oeuvre des différentes techniques (classique, resurfaçage, et semi-resurfaçage), qui préserve le capital osseux du patient et qui soit simple et rapide à poser.

A cet effet, la présente invention se rapporte à une cupule de prothèse, notamment de prothèse d'épaule, comportant une coque ayant sensiblement une forme de calotte sphérique creuse délimitant une surface intérieure concave, la coque présentant un plot d'accouplement s'étendant depuis cette surface intérieure et conçu pour permettre l'accouplement de la cupule avec un élément de fixation, notamment une vis d'ancrage ou une quille d'ancrage, la cupule étant caractérisée en ce qu'elle comprend en outre des moyens d'ancrage et/ou d'accouplement comportant une paroi s'étendant depuis la surface intérieure de la coque et présentant une forme tubulaire, délimitant avec le plot d'accouplement un logement ayant une forme de couronne.

Ainsi, une cupule selon l'invention est modulable : Le plot d'accouplement permet suivant les besoins du chirurgien, d'accoupler la cupule à un élément de fixation. Les moyens d'ancrage et/ou d'accouplement permettent, d'une part, d'ancrer directement dans l'os la cupule pour améliorer sa fixation et, d'autre part, suivant les besoins, d'accoupler la cupule avec un organe intermédiaire présentant une forme de portion de couronne et adapté pour se loger dans le logement délimité entre les moyens d'ancrage et/ou d'accouplement et le plot d'accouplement. Un tel organe intermédiaire peut être par exemple un organe d'accouplement entre la cupule et une vis d'ancrage ou encore une bague intermédiaire permettant d'espacer la cupule de l'os pour une meilleure adaptation de la prothèse sur le patient. Les moyens d'ancrage et/ou d'accouplement permettent donc au chirurgien de choisir la technique qu'il va utiliser pour fixer la cupule sur le patient et éventuellement de changer en cours d'opération. Compte tenu de la compatibilité possible entre la quille d'ancrage et l'organe intermédiaire, le chirurgien a également la possibilité d'utiliser les deux en même temps.

Le logement en forme de couronne, délimité par la paroi tubulaire permet un montage et une fixation simple et rapide d'un organe intermédiaire, tel qu'une bague permettant d'augmenter la hauteur de la couronne. Pour faciliter la fixation d'un organe intermédiaire, la couronne peut former un cône morse.

Selon une caractéristique de l'invention, la paroi est crénelée. Une paroi crénelée permet d'améliorer l'ancrage de la cupule dans l'os lorsque les moyens d'ancrage et/ou d'accouplement sont utilisés pour ancrer la cupule. Le crénelage permet également d'éviter la rotation de la cupule après son insertion dans l'os. Enfin, la paroi crénelée-empêche la rotation d'un organe intermédiaire éventuellement utilisé.

Selon une forme d'exécution, la paroi présente une épaisseur diminuant en éloignement de la surface intérieure. La diminution de l'épaisseur de la paroi en éloignement de la surface intérieure confère à la paroi une capacité de pénétration dans l'os améliorée.

Selon une forme d'exécution, la paroi de forme sensiblement tubulaire présente au moins une rainure longitudinale. Une telle rainure longitudinale permet d'améliorer l'ancrage de la cupule dans l'os.

Selon une forme d'exécution, le logement est configuré pour accueillir un organe intermédiaire présentant une surface extérieure sensiblement tronconique.

Selon une forme d'exécution, le plot d'accouplement et les moyens d'ancrage et/ou d'accouplement sont concentriques, le plot d'accouplement s'étendant à partir du pôle de la coque.

Selon une possibilité, la coque comporte en outre au moins une nervure d'ancrage ménagée sur la surface intérieure. Chaque nervure d'ancrage augmente l'ancrage de la cupule dans l'os. En particulier, lorsque la cupule est accouplée à un organe d'accouplement et que les moyens d'ancrage et/ou d'accouplement ne sont pas utilisés pour ancrer la cupule, chaque nervure d'ancrage assure la fixation de celle-ci. Chaque nervure d'ancrage permet également d'éviter la rotation de la cupule une fois celle-ci mise en place sur un patient et apporte une résistance à l'arrachement en traction à la cupule une fois implantée.

Selon une forme d'exécution, chaque nervure d'ancrage est configurée pour induire une rotation de la cupule lors de sa mise en place sur un os. Ainsi, la rotation de la cupule lors de sa mise en place permet d'améliorer la pénétration des moyens d'ancrage et/ou d'accouplement dans l'os, lorsque ceux-ci sont utilisés pour fixer la cupule. Une fois la cupule implantée, chaque nervure d'ancrage empêche la rotation de la cupule et s'oppose à l'arrachement de la cupule.

Selon une possibilité, la coque présente en outre au moins une encoche disposée sur le bord de la coque. Une telle encoche est utile pour fixer sur la cupule un ancillaire destiné à maintenir la cupule lors de l'impaction. De préférence ledit ancillaire est conçu pour imprimer une rotation à la cupule de 10 degrés en coopérant avec lesdites encoches..

La présente invention concerne enfin un kit comprenant au moins une cupule selon l'invention et au moins un élément de fixation, notamment une vis d'ancrage ou une quille d'ancrage et/ou au moins un organe intermédiaire présentant une forme de portion de couronne et adapté pour se loger dans le logement délimité entre les moyens d'ancrage et/ou d'accouplement et le plot d'accouplement.

L'invention sera bien comprise à l'aide de la description détaillée qui est exposée ci-dessous en regard du dessin annexé, représentant à titre d'exemple non limitatif une forme de réalisation d'une cupule, dans lequel :
La figure 1 est une vue schématique en perspective d'une cupule ;
la figure 2 est une vue schématique en perspective de la cupule de la figure 1 accouplée avec une quille d'ancrage ;
la figure 3 est une vue schématique en perspective de la cupule de la figure 1 accouplée avec une bague intermédiaire et avec une quille d'ancrage ;
la figure 4 est une vue schématique en perspective de la cupule de la figure 1, d'un organe d'accouplement et d'une vis d'ancrage pour la fixation de la prothèse.

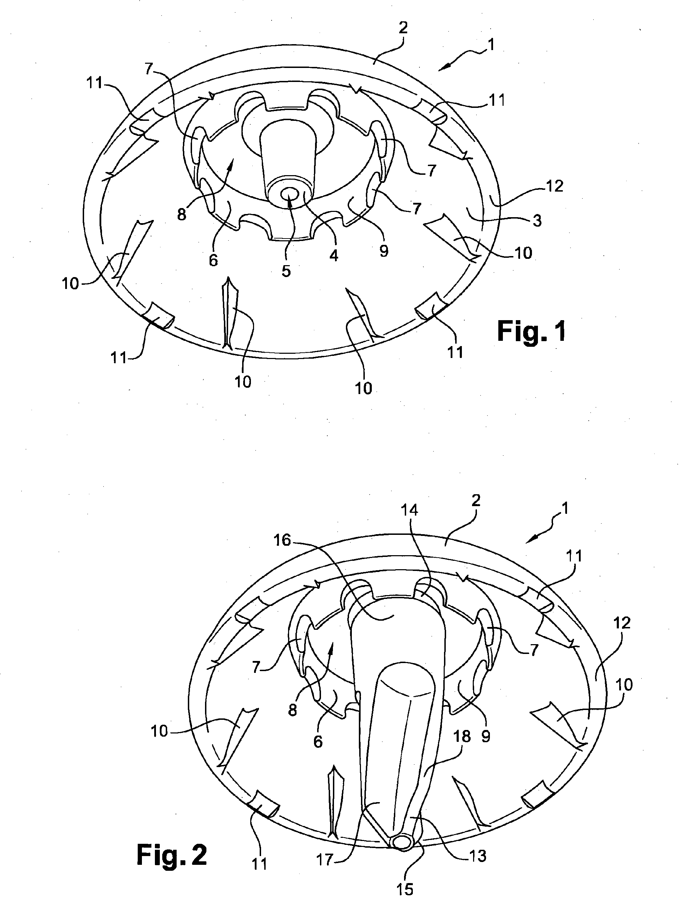

Une cupule 1 de prothèse, illustrée à la figure 1, comporte une coque 2 ayant sensiblement une forme de calotte sphérique creuse. La coque 2 délimite une surface intérieure 3 concave et présente un plot d'accouplement 4 qui s'étend depuis cette surface intérieure 3 au niveau du pôle de la coque 2. Le plot d'accouplement 4 est, par exemple, muni d'un orifice traversant 5 s'étendant suivant l'axe de la calotte sphérique. La cupule 1 comporte en outre des moyens d'ancrage et/ou d'accouplement tels qu'une paroi 6 qui s'étend depuis la surface intérieure 3 et qui présente une forme sensiblement tubulaire. Cette paroi 6 comporte huit créneaux 7 ménagés à l'extrémité libre de celle-ci. Il est bien évident que le nombre de créneaux 7 peut être adapté en fonction des besoins ou de la taille de la cupule 2. De même la forme de chaque créneau 7 est adaptable et peut être, par exemple, arrondie, comme illustrée à la figure 1, ou rectangulaire. Bien entendu, la profondeur de chaque créneau 7 peut varier jusqu'à être sensiblement égale à la hauteur de la paroi 6. L'épaisseur de la paroi 6 diminue en éloignement de la surface intérieure 3. La paroi 6 est disposée de façon concentrique avec le plot d'accouplement 4, de sorte qu'ils délimitent un logement 8 ayant sensiblement une forme de couronne. La face 9 de la paroi 6 disposée en regard du plot d'accouplement 4 présente une forme tronconique femelle.

La cupule 1, illustrée à la figure 1, comporte également huit nervures d'ancrage 10 ménagées sur la surface intérieure 3. Il est bien évident que le nombre de nervures d'ancrage 10 peut être adapté en fonction des besoins ou de la taille de la cupule 2. Chaque nervure d'ancrage 10 s'étend selon un axe longitudinal propre, désaxé par rapport à l'axe de la calotte sphérique.

Enfin, la cupule 1, illustrée à la figure 1, comporte par exemple quatre encoches 11 disposées sur le bord 12 de la coque 2.

Un chirurgien peut implanter la cupule 1 de plusieurs manières détaillées ci-après, suivant ses besoins.

Le chirurgien a tout d'abord la possibilité d'utiliser une quille 13 d'ancrage, illustrée à la figure 2, pour implanter la cupule 1, par exemple dans le cadre d'un resurfaçage de l'épiphyse de l'os.

La quille 13 d'ancrage s'étend suivant un axe longitudinal entre une première extrémité 14, destinée à coopérer avec la cupule 1, et une deuxième extrémité 15, destinée à coopérer avec l'os. Elle présente une forme générale tronconique. La quille 13 d'ancrage comporte une première portion 16 conçue pour permettre l'accouplement de la cupule 1. Cette première portion 16 présente un orifice d'accouplement, non représenté, s'étendant selon l'axe longitudinal de la quille 13 et débouchant sur la première extrémité 14. La forme de cet orifice d'accouplement est complémentaire de la forme du plot d'accouplement 4 de la cupule 1, de manière à permettre un emboitement de celui-ci dans l'orifice d'accouplement de la quille 13 pour accoupler la cupule 1 et la quille 13. De façon avantageuse, le plot d'accouplement 4 et l'orifice d'accouplement sont tronconiques. Par exemple, le plot d'accouplement 4 forme un cône morse mâle et l'orifice d'accouplement est un cône morse femelle complémentaire.

La quille 13 comporte également une deuxième portion 17 conçue pour ancrer la quille 13 dans un os. Cette deuxième portion 17 comporte à cet effet trois arrêtes hélicoïdales 18 permettant à la quille 13 de pénétrer dans l'os à la manière d'un foret. Les arrêtes hélicoïdales 18 impriment un mouvement de rotation à la quille 13 lorsqu'elles pénètrent dans l'os, assurant un meilleur ancrage. Les arrêtes hélicoïdales 18 et les nervures 10 sont conformées pour imprimer une rotation de la cupule 1 dans un même sens : lors de l'impaction une synergie apparait entre les arrêtes hélicoïdales 18 et les nervures 10 qui induisent un mouvement de rotation de l'ordre de 10 degrés.

Lorsque la quille 13 d'ancrage est utilisée pour fixer la cupule 1, le chirurgien accouple la cupule 1 sur la quille 13, en insérant le plot d'accouplement 4 dans l'orifice d'accouplement de la quille 13, puis fixe la quille 13 dans l'épiphyse de l'os. L'impaction de la quille 13 et de la cupule 2 est poursuivie jusqu'à ce que la paroi 6 des moyens d'ancrage et/ou d'accouplement soit enfoncée dans l'épiphyse de l'os pour assurer un ancrage supplémentaire de la cupule 1.

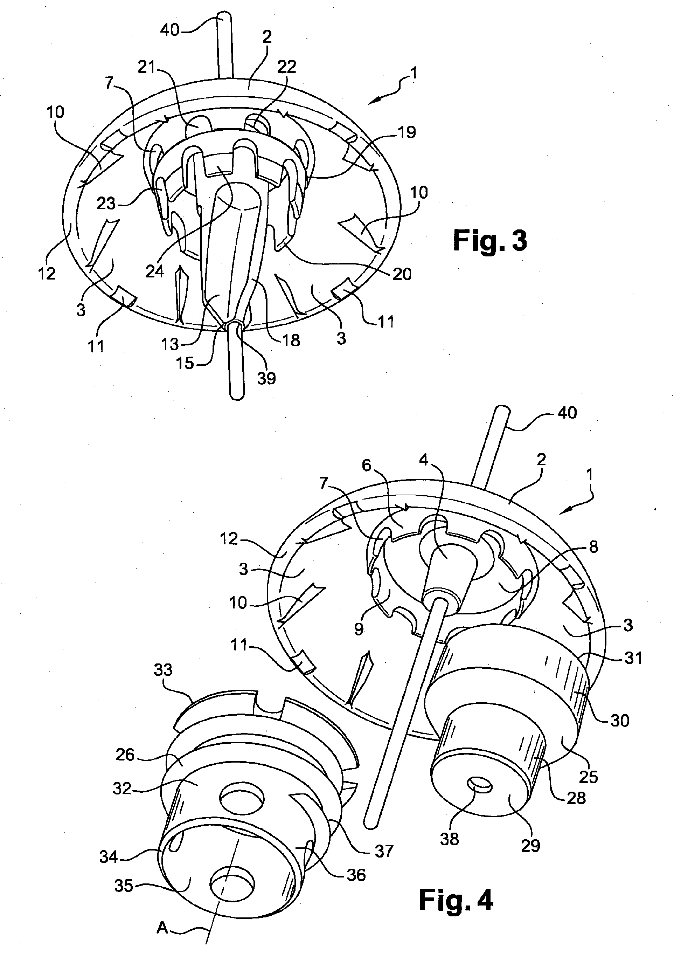

La paroi 6 présente une hauteur n'excédant pas 10% du rayon de la calotte sphérique. De préférence une la paroi 6 présente une hauteur inférieure à 7 mm. Lorsque l'épiphyse de l'os a été partiellement ou totalement réséquée, la hauteur de la paroi 6 peut s'avérer insuffisante pour que les moyens d'ancrage et/ou d'accouplement soient ancrés dans l'os. Le chirurgien a alors la possibilité d'utiliser une bague intermédiaire 19 illustrée à la figure 3. Une telle bague intermédiaire 19 présente un corps tubulaire 20 s'étendant selon un axe longitudinal entre deux extrémités opposées. Le corps tubulaire 20 comprend une portion d'accouplement 21 ayant une forme adaptée pour être insérée dans le logement 8 de la cupule 1, de sorte que la surface extérieure 22 de cette portion d'accouplement 21 soit en contact avec la face 9 de la paroi 6 disposée en regard du plot d'accouplement 4 pour assurer un accouplement sans jeu de la cupule 1 et de la bague intermédiaire 19. La portion d'accouplement 21 peut éventuellement comporter une butée, non représentée, faisant saillie de la surface extérieure 22, ladite butée étant destinée à coopérer avec un des créneaux 7 de la cupule 1 pour bloquer la rotation de la bague intermédiaire 19 dans le logement 8.

La bague intermédiaire 19 présente également une portion d'ancrage 23 comportant une paroi d'extension 24 ayant une forme semblable à la paroi 6. Ainsi la paroi d'extension 24 constitue un prolongement de la paroi 6 et est destinée à être fixée dans l'os de la même manière que la paroi 6. Lorsque la bague intermédiaire 19 est accouplée avec la cupule 1, l'extrémité libre de la bague intermédiaire 19 se situe à une distance du pôle de la calotte sphérique comprise entre 90% et 100% du rayon de celle-ci, de sorte que l'extrémité libre de la bague intermédiaire 19 affleure le bord 12 de la coque 2. Ainsi, l'ablation totale de la cupule 1 est possible et simple puisqu'il suffit de scier l'os à l'aide d'une scie guidée par le bord 12 de la coque 2 : la bague intermédiaire 19 n'est alors pas sectionnée par la scie et peut être entièrement ôtée avec la cupule 1.

Le chirurgien peut également utiliser un organe d'accouplement 25 et une vis 26 d'ancrage pour la fixation de la cupule, illustrés à la figure 4.

L'organe d'accouplement 25 comporte une première portion 27 de forme sensiblement tronconique présentant une portion de montage intermédiaire mâle 28 formant un cône morse mâle 29. L'organe d'accouplement 25 comporte également une deuxième portion tubulaire 30 munie d'une cavité, non représentée, sensiblement tronconique délimitant une portion de montage intermédiaire femelle. La portion de montage intermédiaire femelle forme un cône morse femelle, non représenté, dimensionné pour coopérer par complémentarité de forme avec le plot d'accouplement 4 de la cupule 1. Selon la forme d'exécution représentée sur les figures, l'organe d'accouplement 25 présente un cône morse mâle 29 et un cône morse femelle dont les axes de symétrie respectifs sont confondus. De façon alternative et non représentée, le cône morse mâle 29 et le cône morse femelle peuvent présenter des axes de symétries respectifs distincts décalés et/ou non parallèles. La surface extérieure 31 de la deuxième portion tubulaire 30 a une forme adaptée pour être insérée dans le logement 8 de la cupule 1, de sorte que la surface extérieure 31 de cette deuxième portion tubulaire 30 soit en contact avec la face 9 de la paroi 6 disposée en regard du plot d'accouplement 4 pour assurer un accouplement sans jeu de la cupule 1 et de l'organe d'accouplement 25.

La vis 26 d'ancrage, illustrée à la figure 4, comporte un corps tubulaire 32 s'étendant selon un axe longitudinal A entre une extrémité proximale 33, destinée à coopérer, directement ou indirectement, avec la cupule 1 ou l'organe d'accouplement 25, et une extrémité distale 34 opposée, conçue pour être fixée dans l'épiphyse de l'os. Le corps tubulaire 32 délimite une surface périphérique intérieure 35, illustrée à la figure 4, et une surface périphérique extérieure 36 pourvue de moyens de vissage, réalisés, par exemple, sous la forme d'un filetage 37 pour permettre le vissage de la vis 26 dans l'os.

La surface périphérique intérieure 35 délimite une portion de montage femelle, non représentée, qui s'étend sensiblement à partir de l'extrémité proximale 33 et qui est dimensionnée pour coopérer par complémentarité de forme avec le cône morse mâle 29 de l'organe d'accouplement 25. Dans une forme d'exécution non représentée sur les figures, la portion de montage femelle est dimensionnée pour coopérer par complémentarité de forme avec le plot d'accouplement 4 de la cupule 1 et pour être directement insérée dans le logement 8.

Ainsi, la vis 26 d'ancrage décrit ci-dessus permet de fixer la cupule 1. Lorsque le corps tubulaire 32 de la vis 26 est fixé dans l'os, l'ancrage correct de la vis 26 est assuré grâce au filetage 37 et la procédure de pose est simplifiée, puisque l'accouplement de la cupule 1 sur la vis 26 directement ou indirectement avec l'organe d'accouplement 25 s'effectue par simple emboitement.

Afin de faciliter le positionnement de la cupule 1 et de la quille 13 d'ancrage ou de l'organe d'accouplement 25, ces derniers comportent chacun un orifice traversant 38, 39 s'étendant selon l'axe longitudinal respectivement de l'organe d'accouplement 25 ou de la quille d'ancrage 13. Ainsi, quelque soit la méthode utilisée pour implanter la cupule 1, le chirurgien a la possibilité d'utiliser une tige 40, illustrée aux figures 3 et 4, pour aligner la cupule 1 et la quille d'ancrage 13 ou l'organe d'accouplement 25 sur l'épiphyse. La tige 40 est d'abord insérée dans un orifice ménagé à cet effet dans l'os pour guider et garantir l'orientation correcte de la cupule 1. La quille d'ancrage 13 ou l'organe d'accouplement 25 et la cupule sont ensuite simplement enfilés sur la tige 40 grâce à leurs orifices traversants 5, 39, 38 respectifs.

Ainsi, la cupule 1 selon l'invention décrit ci-dessus présente une grande adaptabilité aux différentes techniques chirurgicales couramment utilisées pour la pose d'une prothèse d'épaule. Les moyens d'ancrage et/ou d'accouplement permettent de fixer directement dans l'os la cupule 1. Ils permettent également, suivant les besoins du chirurgien, d'accoupler la cupule 1 à un organe d'accouplement 25 ou à une vis 26 d'ancrage présentant une forme de portion de couronne adaptée pour se loger dans le logement 8 délimité entre les moyens d'ancrage et/ou d'accouplement et le plot d'accouplement 4.

Enfin, la cupule 1, la quille 13 d'ancrage, l'organe d'accouplement 25, la bague intermédiaire 19 et/ou la vis 26 sont avantageusement recouverts, en totalité ou en partie d'une couche de matériau favorisant l'intégration osseuse et la repousse osseuse telle que, par exemple, une couche d'hydroxyapatite.

Bien entendu, l'exemple de réalisation évoqué ci-dessus ne présente aucun caractère limitatif et d'autres détails et améliorations peuvent être apportés à la cupule 1 selon l'invention, sans pour autant sortir du cadre de l'invention où d'autres formes de cupule peuvent être réalisées.

## Revendications

1. Cupule (1) de prothèse, notamment de prothèse d'épaule, comportant une coque (2) ayant sensiblement une forme de calotte sphérique creuse délimitant une surface intérieure (3) concave, la coque (2) présentant un plot d'accouplement (4) s'étendant depuis cette surface intérieure (3) et conçu pour permettre l'accouplement de la cupule (1) avec un élément de fixation, notamment une vis (26) d'ancrage ou une quille (13) d'ancrage, la cupule (1) étant **caractérisée en ce qu'**elle comprend en outre des moyens d'ancrage et/ou d'accouplement comportant une paroi (6) s'étendant depuis la surface intérieure (3) de la coque (2) et présentant une forme tubulaire, délimitant avec le plot d'accouplement (4) un logement (8) ayant une forme de couronne.

2. Cupule (1) selon la revendication 3, **caractérisée en ce que** la paroi (6) est crénelée.

3. Cupule (1) selon l'une des revendications 1 ou 2, **caractérisée en ce que** la paroi (6) présente une épaisseur diminuant en éloignement de la surface intérieure (3).

4. Cupule (1) selon l'une des revendications 1 à 4, **caractérisée en ce que** le logement (8) est configuré pour accueillir un organe intermédiaire présentant une surface extérieure sensiblement tronconique.

5. Cupule (1) selon l'une des revendications 1 à 5, **caractérisée en ce que** le plot d'accouplement (4) et les moyens d'ancrage et/ou d'accouplement sont concentriques, le plot d'accouplement (4) s'étendant à partir du pôle de la coque (2).

6. Cupule (1) selon la revendication 5, **caractérisée en ce que** la coque (2) comporte en outre au moins une nervure (10) d'ancrage ménagée sur la surface intérieure (3).

7. Cupule (1) selon la revendication 6, **caractérisée en ce que** chaque nervure (10) d'ancrage est configurée pour induire une rotation de la cupule (1) lors de sa mise en place sur un os.

8. Cupule (1) selon l'une des revendications 1 à 7, **caractérisée en ce que** la coque (2) présente en outre au moins une encoche (11) disposée sur le bord (12) de la coque (2).

## Patentansprüche

1. Prothesenpfanne (1), insbesondere für eine Schulterprothese, die eine Schale (2) umfasst, die im Wesentlichen die Form einer hohlen Kugelkappe aufweist, die eine konkave Innenoberfläche (3) begrenzt, wobei die Schale (2) ein Kopplungsstück (4) aufweist, das sich ausgehend von der Innenoberfläche (3) erstreckt und ausgestaltet ist, das Koppeln der Pfanne (1) mit einem Fixierungselement, insbesondere mit einer Verankerungsschraube (26) oder mit einem Verankerungskiel (13) oder Verankerungskegel (13), zu ermöglichen, wobei die Pfanne (1) **dadurch gekennzeichnet ist, dass** sie überdies Verankerungsmittel und/oder Kopplungsmittel umfasst, die eine Wand (6) umfassen, die sich ausgehend von der Innenoberfläche (3) der Schale erstreckt und die eine Röhrenform aufweist, die mit dem Kopplungsstück (4) eine Aufnahme (8) begrenzt, die die Form einer Krone oder eines Kranzes aufweist.

2. Pfanne (1) nach Anspruch 1, **dadurch gekennzeichnet, dass** die Wand (6) gezackt ist.

3. Pfanne (1) nach einem der Ansprüche 1 oder 2, **dadurch gekennzeichnet, dass** die Wand (6) eine Stärke aufweist, die mit Entfernung von der Innenoberfläche (3) abnimmt.

4. Pfanne (1) nach einem der Ansprüche 1 bis 3, **dadurch gekennzeichnet, dass** die Aufnahme (8) ausgestaltet ist, ein Zwischenelement aufzunehmen, das eine im Wesentlichen kegelstumpfförmige Außenoberfläche aufweist.

5. Pfanne (1) nach einem der Ansprüche 1 bis 4, **dadurch gekennzeichnet, dass** das Kopplungsstück (4) und die Verankerungsmittel und/oder Kopplungsmittel konzentrisch sind, wobei das Kopplungsstück (4) sich vom Pol der Schale (2) aus erstreckt.

6. Pfanne (1) nach Anspruch 5, **dadurch gekennzeichnet, dass** die Schale (2) überdies mindestens eine Verankerungsrippe (10) umfasst, die auf der Innenoberfläche (3) angeordnet ist.

7. Pfanne (1) nach Anspruch 6, **dadurch gekennzeichnet, dass** jede Verankerungsrippe (10) ausgestaltet ist, beim Anbringen auf einem Knochen eine Rotation der Pfanne (1) zu bewirken.

8. Pfanne (1) nach einem der Ansprüche 1 bis 7, **dadurch gekennzeichnet, dass** die Schale (2) überdies mindestens eine Einkerbung (11) aufweist, die auf dem Rand (12) der Schale (2) angeordnet ist.

## Claims

1. Prosthesis cup (1), particularly a shoulder prosthesis cup, comprising a shell (2) substantially shaped as a hollow spherical cap delimiting a concave inner surface (3), the shell (2) having a coupling stem (4) that extends from this inner surface (3) and is designed to permit coupling of the cup (1) to a fixing element, particularly an anchoring screw (26) or an anchoring pin (13), the cup (1) being **characterized in that** it additionally comprises anchoring and / or coupling means with a wall (6) which extends from the inner surface (3) of the shell (2) and has a substantially tubular shape and which, together with the coupling stem (4), delimits a recess (8) having a ring shape.

2. Cup (1) according to claim 3, **characterized in that** the wall (6) is serrated.

3. Cup (1) according to one of claims 1 or 2, **characterized in that** the wall (6) has a thickness that decreases in a direction going away from the inner surface (3).

4. Cup (1) according to one of claims 1 to 4, **characterized in that** the recess (8) is configured to receive an intermediate member having a substantially frusto-conical outer surface.

5. Cup (1) according to one of claims 1 to 5, **characterized in that** the coupling stem (4) and the anchoring and / or coupling means are concentric, the coupling stem (4) extending from the pole of the shell (2).

6. Cup (1) according to claim 5, **characterized in that** the shell (2) also includes at least one anchoring rib (10) formed on the inner surface (3).

7. Cup (1) according to claim 6, **characterized in that** each anchoring rib (10) is configured to cause the cup (1) to rotate when it is placed on a bone.

8. Cup (1) according to one of claims 1 to 7, **characterized in that** the shell (2) also has at least one notch (11) arranged on the edge (12) of the shell (2).
